# EUROPÄISCHE PATENTSCHRIFT

(11) **EP 2 841 123 B1**
(45) Veröffentlichungstag und Bekanntmachung des Hinweises auf die Patenterteilung: **06.07.2016**
(21) Anmeldenummer: 13719515.2
(22) Anmeldetag: 25.04.2013
(51) Int. Cl.: A61M 1/10

(54) **PULSATIONSBLUTPUMPE**
PULSATILE BLOOD PUMP
POMPE À SANG À PULSATIONS

(30) Priorität: 27.04.2012 DE 102012207042
(43) Veröffentlichungstag der Anmeldung: 04.03.2015
(73) Patentinhaber: Abiomed Europe GmbH, 52074 Aachen (DE)
(72) Erfinder: SPANIER, Gerd, 52074 Aachen (DE)
(74) Vertreter: Klunker . Schmitt-Nilson . Hirsch
(86) Internationale Anmeldenummer: PCT/EP2013/058648
(87) Internationale Veröffentlichungsnummer: WO 2013/160411

(56) Entgegenhaltungen:
- WO-A2-2004/078025
- WO-A2-2007/089500
- US-A1- 2005 096 496
- US-A1- 2010 036 486
- US-A1- 2010 268 333

## Beschreibung

Die Erfindung betrifft eine extravasale Pulsationsblutpumpe und findet Anwendung bei der Unterstützung der Pulsation des menschlichen Herzens.

Um es einem vorgeschädigten menschlichen Herzen zu ermöglichen, sich zu erholen, wird die Pulsation des Herzens mittels künstlicher Pumpen unterstützt. Breite Anwendung finden sogenannte intraaortale Ballonpumpen (IABP). Dazu wird ein Ballon in der Aorta platziert und von außerhalb des Körpers durch einen relativ langen Katheter hindurch abwechselnd, entsprechend dem Herzrythmus, mit Helium gefüllt und entleert. Das Entleeren erfolgt kurz vor Beginn der Systole, um auf diese Weise den Blutdruck in der Aorta signifikant zu senken, so dass das Herz sein Blutvolumen gegen einen geringen Aortendruck in die Aorta ausstoßen kann. Am Anfang der Diastole wird der Ballon wieder gefüllt, erhöht dadurch den Druck in der Aorta und treibt so das Blut in die Organe und peripheren Blutbahnen. Dieses Verfahren ist auch als aortale Gegenpulsation bzw. Counterpulsation-Verfahren bekannt.

In US 2005/0096496 A1 werden verschiedene Nachteile von intraaortalen Ballonpumpen herausgestellt. Unter anderem sind sie wegen des ständig liegenden Katheters infektionsbehaftet und erlauben es dem Patienten nicht, das Bett zu verlassen. Daher werden sie in der Regel nur über ein oder zwei Tage eingesetzt, obwohl chronisch schwache Herzen eine langfristige Unterstützung benötigen würden. Außerdem behindert der Katheter in der Blutbahn den Blutfluss. Es wird dort daher vorgeschlagen, eine subkutan implantierte Pumpe über eine Leitung an eine arterielle Blutbahn anzuschließen und Blut aus der Arterie abzusaugen, wenn das Herz kontrahiert (Systole), und wieder in die Arterie zurückzupumpen, wenn das Herz erschlafft (Diastole). Auf diese Weise wird eine Gegenpulsation erreicht, ohne dass ein Ballon die Blutbahn selbst verschließt oder ein Katheter innerhalb der Blutbahn verläuft. Die Pumpe kann als Blase, Sack oder Diaphragmapumpe ausgebildet sein. Es wird auch vorgeschlagen, die extravasale Gegenpulsationspumpe durch eine zweite extravasale Pumpe zu ergänzen, mit der Blut kontinuierlich über eine erste Leitung direkt aus dem Herzen abgesaugt und über eine zweite Leitung unmittelbar derjenigen Arterie zugeführt wird, an die auch die extravasale Gegenpulsationspumpe angeschlossen ist.

Mit extravasalen Gegenpulsationsblutpumpen sind wesentlich höhere Volumenströme erzielbar als mit herkömmlichen aortalen Ballonpumpen, nämlich bis zu 2,4 l pro Minute anstelle von 0,71 pro Minute. Derartige Gegenpulsationssysteme lassen sich darüber hinaus durch Copulsationssysteme ersetzen oder ergänzen, bei denen die Leitung der extravasalen Blutpumpe unmittelbar mit einem Ventrikel des Herzens verbunden wird. Die Pumpe saugt dann während der Diastole temporär Blut aus dem Ventrikel in eine Kammer, um so das Blutvolumen im Ventrikel klein zu halten und ein Dilatieren des Herzens zu verhindern, und pumpt dieses Blut während der anschließenden Systole in den Ventrikel zurück, von wo aus es durch die geöffnete Herzklappe in die arterielle Blutbahn strömt.

Sowohl die Gegenpulsation als auch die Copulsation mittels einer im Körper vollimplantierten extravasalen Blutpumpe besitzen das Problem, dass das mittels der Pumpe abgesaugte Blut in einem Speicher zwischengespeichert werden muss. Dazu besitzt die Blutpumpe eine sogenannte ComplianceKammer deren Volumen sich beim Füllen der Blutpumpe entsprechend verringert. Compliance-Kammern sind relativ voluminös. Dies gilt insbesondere für gasgefüllte Compliance-Kammern, weil die Pumpe bei einer kleinen gasgefüllten Compliance-Kammer viel Energie benötigen würde, um das Gasvolumen der Compliance-Kammer während der Ansaugphase entsprechend zu komprimieren. Dieses Problem verdoppelt sich, wenn sowohl eine Gegenpulsationspumpe als auch eine Copulsationspumpe gleichzeitig implantiert werden.

US 2010/0268333 A1 beschreibt verschiedene Systeme und Verfahren zur Steuerung von Blutpumpen, wobei einzelne der dort beschriebenen Blutpumpen bauartbedingt auch rückwärts betrieben werden können und insofern als bidirektionales Pumpsystem bezeichnet werden können. Es wird dort vorgeschlagen, die beschriebenen Pumpen zur Herzunterstützung als eine Art Bypass zwischen dem linken Ventrikel und der aufsteigenden oder absteigenden Aorta einzusetzen und das Blut an der Aortenklappe vorbei unmittelbar vom linken Ventrikel in die Aorta zu fördern. Dies kann wiederum im Copulsationsmodus erfolgen, also während der Kontraktionsphase des Herzens (Systole), indem die Pumpe den Großteil oder den gesamten Blutfluss übernimmt, während der Ventrikel nur mit jedem vierten oder fünften Herzschlag durch die Aortenklappe fördert, um einen Strömungsstillstand an der Aortenwurzel zu vermeiden, der ansonsten zu Thrombosekomplikationen führen könnte. Alternativ kann das Blut während der Erschlaffungsphase des Herzens (Diastole) vom linken Ventrikel in die Aorta gepumpt werden, nämlich im so genannten Gegenpulsationsmodus, bei dem ein wesentlicher Teil des Blutes nach wie vor vom Ventrikel durch die Aortenklappe in die Aorta gefördert wird.

Aufgabe der vorliegenden Erfindung ist es, die Unterstützung der Pulsation des Herzens mittels extravasaler Pulsationsblutpumpen zu verbessern und insbesondere eine Pulsationsblutpumpe vorzuschlagen, die hinsichtlich ihrer Funktionalität und Baugröße im Vergleich zu den vorbeschriebenen extravasalen Pulsationsblutpumpen optimiert ist.

Diese Aufgabe wird durch eine Pulsationsblutpumpe mit den Merkmalen des Anspruchs 1 gelöst. In davon abhängigen Ansprüchen sind vorteilhafte Ausgestaltungen und Weiterbildungen der Erfindung angegeben.

Die erfindungsgemäße Pulsationsblutpumpe kombiniert die Funktionen der Gegenpulsation und Copulsation und besitzt zu diesem Zweck ein bidirektional wirkendes Pumpsystem, welches über zwei Leitungen einerseits mit einer Blutbahn, zum Beispiel der Aorta, und andererseits mit einer Herzkammer, zum Beispiel dem linken Ventrikel, verbunden ist. Zwischen dem Herz und der Blutbahn liegt ein Ventil, welches normalerweise durch eine Herzklappe gebildet wird. Das bidirektionale Pumpsystem ist dazu eingerichtet, während der Diastole des Herzens eine erste Menge Blut aus dem Herzen zu entnehmen und im Wesentlichen gleichzeitig eine zweite Menge Blut in die Blutbahn einzubringen. Andererseits ist das Pumpsystem dazu eingerichtet, während der auf die Diastole folgenden Systole des Herzens eine der zweiten Menge entsprechende Menge Blut aus der Blutbahn wieder zu entnehmen und im Wesentlichen gleichzeitig wiederum eine der ersten Menge entsprechende Menge Blut in das Herz einzubringen. Die ersten und zweiten Mengen können identisch sein, aber das ist nicht zwingend, denn das Pumpsystem kann als Differentialpumpsystem ausgebildet sein.

Im Ergebnis wird das Herz sowohl während der Systole als auch während der Diastole deutlich entlastet. Denn während der Systole fördert das Herz gegen einen geringeren arteriellen Druck, weil der Blutbahn, in die das Herz fördert, gleichzeitig Blut entnommen wird. Während der anschließenden Diastole wird das Herz dadurch entlastet, dass ein Teil des Füllvolumens mittels der Blutpumpe aus der betreffenden Herzkammer entnommen und erst während der darauf folgenden Systole wieder in die Herzkammer eingebracht wird. Die Herzkammer dehnt sich somit weniger aus, so dass ein Dilatieren oder Aufweiten der Herzkammer verhindert oder verringert wird. Selbst wenn sich die Herzkammer während der Diastole bis auf ihr normales Maß weitet und mit Blut füllt, so wird mittels der erfindungsgemäßen Pulsationsblutpumpe zumindest erreicht, dass sich das Füllvolumen der betreffenden Herzkammer um die Menge des mittels des bidirektionalen Pumpsystems aus der Herzkammer entnommenen Bluts "vergrößert", weil eben diese Menge während der nachfolgenden Systole wieder in die Herzkammer zurückgeführt wird und diese Menge an Blut zeitgleich zur Herzaktivität in das anschließende Gefäßsystem ausgeworfen wird. Während auf diese Weise das Herz während der Diastole entlastet und/oder kapazitiv erweitert wird, wird der Druck in der zugehörigen Blutbahn, beispielsweise der Aorta, aufgrund der gleichzeitig in die Blutbahn eingebrachten Blutmenge erhöht, so dass aufgrund des erhöhten Blutdrucks in der betreffenden Blutbahn die Organe und angrenzenden Blutgefäße vermehrt mit Blut versorgt werden. Die erfindungsgemäße Pulsationsblutpumpe mit bidirektional wirkendem Pumpsystem kombiniert somit die Funktionen und Vorteile einer copulsierenden extravasalen Blutpumpe mit denen einer gegenpulsierenden extravasalen Blutpumpe.

Ein wesentlicher weiterer Vorteil der erfindungsgemäßen Pulsationsblutpumpe besteht aber darin, dass es nicht notwendig ist, für jede dieser beiden Funktionen eine separate Compliance-Kammer vorzusehen. Statt dessen kann das Pumpsystem beispielsweise eine erste Pumpkammer mit variablem Volumen aufweisen, die zum Beispiel an die Herzkammer angeschlossen ist, und eine zweite Pumpkammer mit variablem Volumen, die dann an die entsprechende Blutbahn angeschlossen ist, wobei die beiden Pumpkammern so miteinander gekoppelt sind, dass in dem Maße, wie Blut in die erste Pumpkammer angesaugt wird, Blut aus der zweiten Pumpkammer ausgestoßen wird, und umgekehrt. Dementsprechend wirken die beiden Pumpkammern für die jeweils andere Pumpkammer als Compliance-Kammer. Dies lässt sich anschaulich mit einem zweiseitig wirkenden Zylinderkolben vergleichen. Während durch Verlagerung des Kolbens innerhalb eines Zylinders das Volumen in Bewegungsrichtung vor dem Kolben verringert wird, vergrößert sich das Volumen hinter dem Kolben. Das sich verringernde Volumen vor dem Kolben ist vergleichbar mit einer Compliance-Kammer für das sich vergrößernde Volumen hinter dem Kolben. Bei umgekehrter Bewegungsrichtung des Kolbens dreht sich diese Funktionalität entsprechend um. Das heißt, die unter Druck stehende Kammer des Kolbenzylinders wirkt immer gleichzeitig als Pumpkammer, aus der heraus gefördert wird, und als Compliance-Kammer für die auf der anderen Seite des Kolbens liegende Kammer.

Dieses grundlegende Prinzip lässt sich in verschiedener Weise abwandeln. Insbesondere lässt sich mittels eines Differentialkolbens ein Differentialpumpsystem realisieren, welches unterschiedliche Volumenströme in die eine und die andere Richtung fördert - bei absolut betrachtet gleicher Kolbenwegstrecke.

Erfindungsgemäß helfen die jeweiligen Drücke auf der Saugseite der Pumpe, die erforderliche Energie zur Verschiebung des Kolbens oder der hydraulischen Flüssigkeit gering zu halten. Dies ist besonders für ein voll implantierbares System entscheidend, um die Batteriegröße gering zu halten.

Eine andere Abwandlung des vorbeschriebenen grundlegenden Prinzips sieht separate Compliance-Kammern vor. Das heißt, während bei dem vorbeschriebenen zweiseitig wirkenden Zylinderkolben das Volumen auf einer Seite des Kolbens gleichzeitig als Pumpkammer und als ComplianceKammer wirkt, sind diese beiden Funktionen bei dieser abgewandelten Ausführungsform voneinander getrennt. So kann das bidirektionale Pumpsystem eine erste Pumpkammer mit variablem Volumen und eine erste Compliance-Kammer mit variablem Volumen besitzen, die gemeinsam eine erste Doppelkammer bilden, und eine zweite Pumpkammer mit variablem Volumen kann mit einer zweiten Compliance-Kammer mit variablem Volumen gemeinsam eine zweite Doppelkammer bilden. Dabei sind die Compliance-Kammern von der jeweils zugehörigen Pumpkammer durch eine variable Trennwand getrennt, die beispielsweise als flexible Membran ausgebildet sein oder zumindest eine flexible Membran umfassen kann. Mittels einer Pumpe wird nun ein Fluid zwischen den beiden Compliance-Kammern hinund hergefördert, so dass je nach Förderrichtung aus der einen zugehörigen Pumpkammer Blut ausgestoßen wird, während gleichzeitig in die andere zugehörige Pumpkammer Blut angesaugt wird, und umgekehrt.

Der Vorteil dieser letztgenannten Abwandlung des grundlegenden Prinzips besteht gegenüber herkömmlichen Systemen vor allem darin, dass die Compliance-Kammern statt mit einem Gas mit einer Flüssigkeit gefüllt sein können, so dass die Compliance-Kammern nicht größer sein müssen als das von den Pumpkammern aufzunehmende Blutvolumen. Dadurch ist das System besonders effizient und darüberhinaus sicherer im Vergleich zu gasgefüllten Compliance-Kammern. Als Flüssigkeit bietet sich jede Flüssigkeit an. Ein weiterer Vorteil dieser Abwandlung gegenüber dem zweiseitig wirkenden Zylinderkolben liegt darin, dass die Pumpe vom Blut entkoppelt ist, das heißt die Pumpe fördert nur Flüssigkeit zwischen den beiden Compliance-Kammern, und kein Blut.

Die erfindungsgemäße Pulsationsblutpumpe kann vollständig in den Körper eines Patienten implantiert werden. Zumindest aber das Pumpsystem ist dazu vorgesehen und eingerichtet, implantiert zu werden. Eine Energieversorgung für die Pumpe oder, falls die Pumpe mittels eines separaten Motors angetrieben wird, für diesen Motor, kann ebenfalls implantierbar sein und beispielsweise transkutan entweder kontaktbehaftet oder vorzugsweise kontaktlos von Zeit zu Zeit oder kontinuierlich aufgeladen werden.

Selbstverständlich umfasst die erfindungsgemäße Pulsationsblutpumpe eine Steuerung, die vorgesehen und dazu eingerichtet ist, das bidirektionale Pumpsystem entsprechend einem vorgegebenen Herzrhythmus abwechselnd in die eine und die andere Richtung zu betreiben. Der Herzrhythmus kann in unterschiedlicher Weise mittels geeigneter Sensorik erfasst und die so ermittelten Herzrhythmusdaten an die Steuerung übermittelt werden. Insbesondere kann die Pulsationsblutpumpe mittels derselben Herzrhythmusdaten gesteuert werden, die auch zur Steuerung herkömmlicher synchroner Herzunterstützungssysteme, wie z.B. intraaortaler Blutpumpen, verwendet werden.

Nachfolgend wird die Erfindung anhand der begleitenden Zeichnungen beispielhaft erläutert. Darin zeigen:
- Figur 1: das grundsätzliche Prinzip einer erfindungsgemäßen Pulsationsblutpumpe am Beispiel der Unterstützung des linken Ventrikels,
- Figur 2: eine erste Abwandlung des grundsätzlichen Prinzips,
- Figur 3: eine zweite Abwandlung des grundsätzlichen Prinzips und
- Figur 4: das grundsätzliche Prinzip am Beispiel der Unterstützung des rechten Ventrikels.

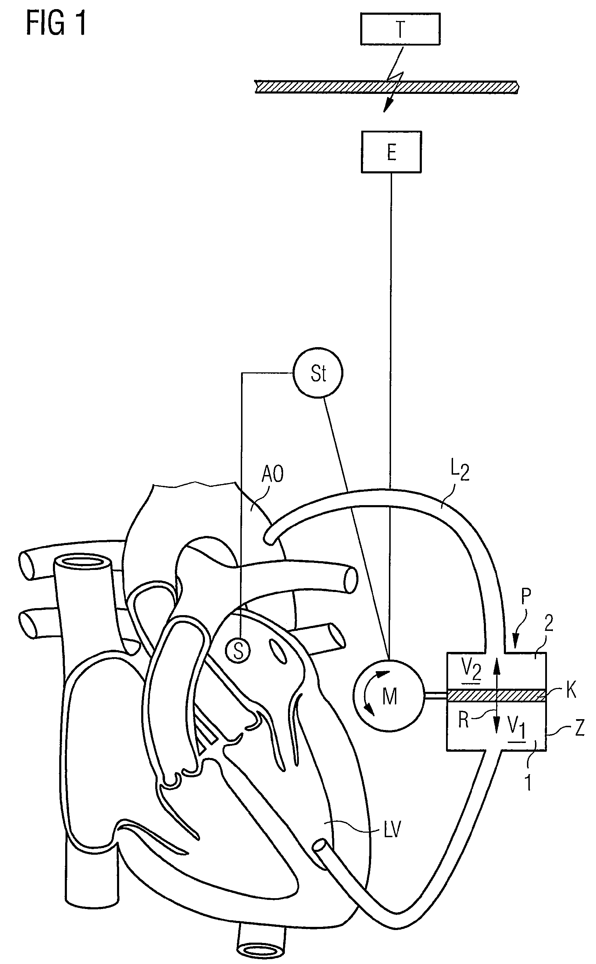

Anhand der schematischen Darstellung gemäß Figur 1 wird nachfolgend das grundsätzliche Prinzip der erfindungsgemäßen Pulsationsblutpumpe erläutert. Die Pulsationsblutpumpe besteht im Wesentlichen aus einem bidirektionalen Pumpsystem, welches über eine erste Leitung L1 und eine zweite Leitung L2 einerseits mit einer Blutbahn, hier der Aorta AO, und andererseits mit einer Herzkammer, hier dem linken Ventrikel LV, verbunden ist. Das bidirektionale Pumpsystem besteht im Wesentlichen aus einer Pumpe P und einem die Pumpe P antreibenden Motor M. Wie die Pumpe und/oder der Motor im Einzelfall konkret beschaffen und miteinander gekoppelt sind, ist für die Erfindung von untergeordneter Bedeutung. Wesentlich für das in Figur 1 dargestellte grundlegende Prinzip ist, dass die Pumpe P nach Art einer zweiseitig wirkenden Kolben-Zylinder-Anordnung ausgebildet ist, bei der der Kolben K innerhalb eines Zylinders Z in einer Richtung hin und zurück bewegt wird. Dadurch ändern sich die Volumina V₁ und V₂ in den einander gegenüberliegenden, durch den Kolben K getrennten Pumpkammern 1 und 2.

Der Motor M und damit die Pumpe P wird über eine Steuerung St entsprechend vorgegebenen Herzrythmus abwechselnd in die eine und die andere Richtung betrieben. Die Herzrythmusdaten zur Steuerung des Pumpsystems (z.B. Druck, EKG, Kontraktion, PPS, etc.) können mittels einer mit der Steuerung St gekoppelten Sensorik S erfasst und an die Steuerung St übermittelt werden. Dies ist in Figur 1 lediglich schematisch angedeutet durch einen im Vorhof des linken Ventrikel LV liegenden Sensor S, der ein Drucksensor sein kann.

Die für den Betrieb des Pumpsystems notwendige Energie kann aus einem Energiespeicher E zur Verfügung gestellt werden, der beispielsweise kontaktlos entweder ständig oder vorzugsweise zeitweise über einen Transmitter T entsprechend aufgeladen wird.

Unter Verwendung dieser Energie und unter Berücksichtigung der von der Steuerung St verarbeiteten Herzrythmusdaten wird nun der Kolben K entsprechend dem Herzrythmus so verlagert, dass während der Systole das Volumen V₁ der Pumpkammer 1 verringert wird und dementsprechend Blut aus der Pumpkammer 1 über die Leitung L₁ in den linken Ventrikel LV gepumpt wird. Dadurch wird gleichzeitig Blut aus der Aorta AO durch die Leitung L₂ hindurch in das sich vergrößernde Volumen V₂ der zweiten Pumpkammer 2 angesaugt. Der linke Ventrikel LV arbeitet dadurch gegen einen verringerten Aortendruck, und auch das aus der Pumpkammer 1 verdrängte Blutvolumen fließt durch den linken Ventrikel LV und die Aortenklappe in die Aorta AO. Während der nachfolgenden Diastole wird der Kolben K in die entgegengesetzte Richtung bewegt, so dass Blut aus dem linken Ventrikel LV durch die Leitung L1 in die Pumpkammer 1 gesaugt und gleichzeitig eine entsprechende Menge Blut aus der zweiten Pumpkammer 2 durch die Leitung L2 in die Aorta AO gefördert wird. Dadurch wird die Dehnung des linken Ventrikels LV gering gehalten und einem Dilatieren des Herzens entgegengewirkt, so dass sich das Herz erholen kann. Gleichzeitig wird durch das in die Aorta AO geförderte Blut der Blutdruck in der Aorta AO so erhöht, dass das Blut zuverlässig in die Organe, also auch in das Herz, und die peripheren Blutbahnen fließt. Durch Verringerung der diastolischen Ventrikelgröße kann die Wandspannung des Herzmuskels gering gehalten werden und so die Blutversorgung des Herzens noch effizienter erfolgen.

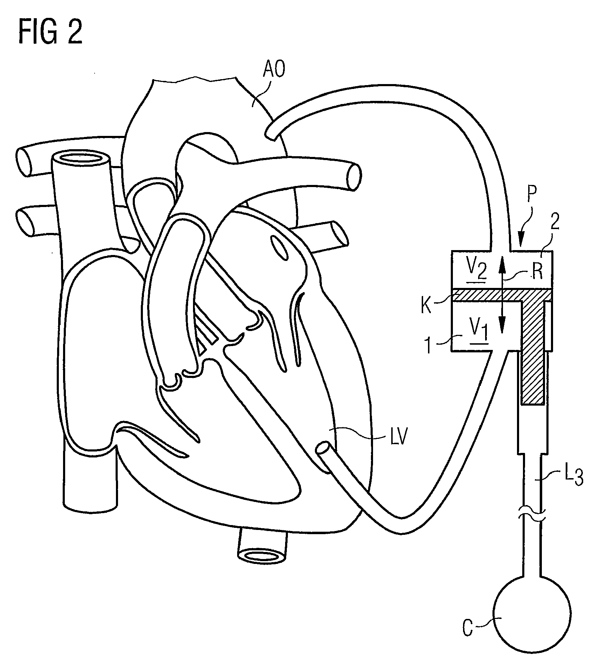

Figur 2 zeigt eine erste Abwandlung dieses grundlegenden Prinzips. Der Kolben K ist hier als Differentialkolben mit zwei unterschiedlich großen Kolbenflächen ausgebildet. Dementsprechend verändern sich die Volumina V₁ und V₂ bei einer Bewegung des Kolbens K in Richtung R nicht im selben Maße. In dem konkret dargestellten Ausführungsbeispiel wird aufgrund des Differentialkolbens K eine geringere Menge Blut zwischen dem linken Ventrikel LV und der Pumpkammer 1 hin und her gefördert als zwischen der Aorta AO und der zweiten Pumpkammer 2. Je nach dem, welche Herzfunktion mit der Pulsationsblutpumpe verstärkt unterstützt werden soll, kann die größere Kolbenfläche des Differentialkolben entweder auf der Seite des Herzens oder der Blutbahn liegen. Der Differentialkolben K benötigt allerdings auf der Seite der kleineren Kolbenfläche eine zusätzliche Compliancekammer C, welche über eine Leitung L₃ mit der Pumpe P verbunden ist. Die Compliancekammer C nimmt die Differenz aus der Volumenverschiebung V₂ und V₁ auf, die je nach Verschieberichtung des Differentialkolbens K positiv oder negativ ist. Die Compliancekammer C wird an einem Ort innerhalb des Patienten platziert, an welchem sie nur einem geringen Umgebungsdruck ausgesetzt ist, beispielsweise im Abdomen, und ist über eine Leitung L₃ mit der Pumpe P verbunden. In der Leitung L₃ und der Compliance-Kammer C befindet sich vorzugsweise eine Flüssigkeit, also insbesondere kein Blut.

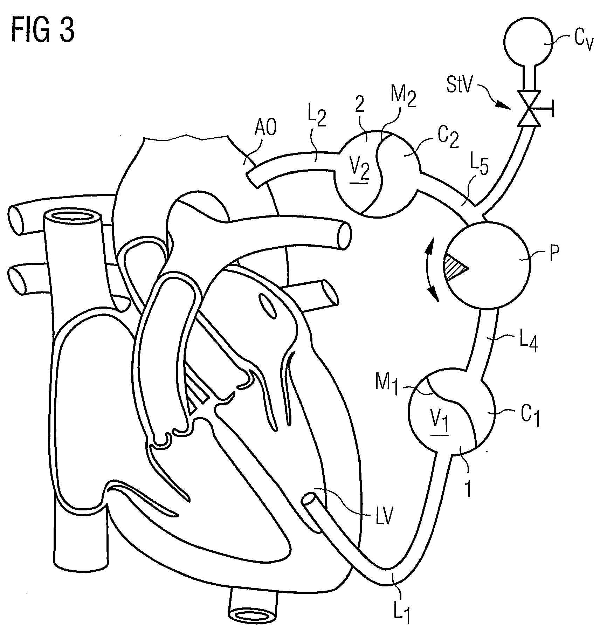

Figur 3 zeigt eine zweite Abwandlung des grundlegenden Prinzips. Hier bildet die erste Pumpkammer 1 mit einer ersten Compliance-Kammer C₁ eine erste Doppelkammer und die zweite Pumpkammer 2 mit einer zweiten Compliance-Kammer C₂ eine zweite Doppelkammer. Die Pumpkammern 1 und 2 sind von den Compliance-Kammern C₁ und C₂ jeweils durch eine Membran M₁ und M₂ getrennt, so dass das Volumen V₁ und V₂ der Pumpkammern 1 und 2 jeweils variabel ist. Mittels einer Pumpe P, die hier nur schematisch wiedergegeben ist und beispielhaft eine bidirektionale Rotationspumpe sein kann, wird dann ein Fluid zwischen den Compliance-Kammern C₁ und C₂ entsprechend dem Herzrythmus so hin- und hergepumpt, dass sich die variablen Volumen V₁ und V₂ der beiden Pumpkammern 1 und 2 in der zuvor in Bezug auf Figur 1 beschriebenen Weise verändern. Dazu ist die Pumpe P über die Leitungen L₄ und L₅ mit den Compliance-Kammern C₁ und C₂ verbunden. In den Leitungen L₄, L₅ und den Compliance-Kammern C₁, C₂ befindet sich ein Hydraulikfluid, also insbesondere kein Blut. Die Pumpe P ist somit vom Blutkreislauf mittels der Membrane M₁ und M₂ zuverlässig abgeschirmt. Der Aufbau und die Effektivität der für das Pumpsystem einsetzbaren Pumpen P ist dadurch begünstigt. Ebenfalls wird dabei die Dauerfestigkeit des Pumpsystems erheblich verbessert.

Eine zusätzliche Compliance-Kammer Cv kann vorgesehen sein, um Volumenschwankungen aufzunehmen, wenn die Blutmengen V₁ und V₂ unterschiedlich groß ausfallen. Figur 3 zeigt eine solche zusätzliche ComplianceKammer Cv zur Aufnahme eines Teils des zwischen den Compliance-Kammern C₁ und C₂ geförderten Fluids. Diese zusätzliche ComplianceKammer Cv ist optional und vorzugsweise bezüglich seiner Compliance-Eigenschaften variabel einstellbar. Zum Einstellen der Compliance-Eigenschaften dient das Steuerventil StV. Die Einstellung kann entweder vor der Implantation oder vorzugsweise zum Beispiel per Remote-Control auch nach der Implantation entweder bedarfsweise oder ständig erfolgen. Dadurch lassen sich die in den Pumpkammern 1 und 2 aufgenommenen Blutvolumina variieren, gegebenenfalls auch dynamisch. Ein Grund für eine solche Maßnahme kann beispielsweise darin liegen, dass sich beim Füllen der einen oder anderen der beiden Pumpkammern 1 und 2 Probleme ergeben oder dass die verfügbaren Volumina der Pumpkammern 1 und 2 absichtlich variiert werden sollen. So ist es möglich, dass trotz der den beiden Pumpkammern 1 und 2 gemeinsamen Pumpe P unterschiedliche Volumina zwischen den zugehörigen Compliance-Kammern C₁ und C₂ gefördert werden, wobei das Differenzvolumen von der zusätzlichen Compliance-Kammer Cv aufgenommen wird. Mittels des Steuerventils StV ist es somit möglich, die Ansaug- und Auswurfvolumina der Pumpkammern 1 und 2 variabel zu steuern. Die Volumenreduzierung darf allerdings nicht dazu führen, dass in einer der Pumpkammern ständig so viel Blut verbleibt, dass sukzessive ein Verklumpen des Bluts zu befürchten ist.

Anstatt die zusätzliche Compliance-Kammer Cv an die Leitung L₅ anzuschließen, kann sie auch an die Leitung L₄ angeschlossen werden.

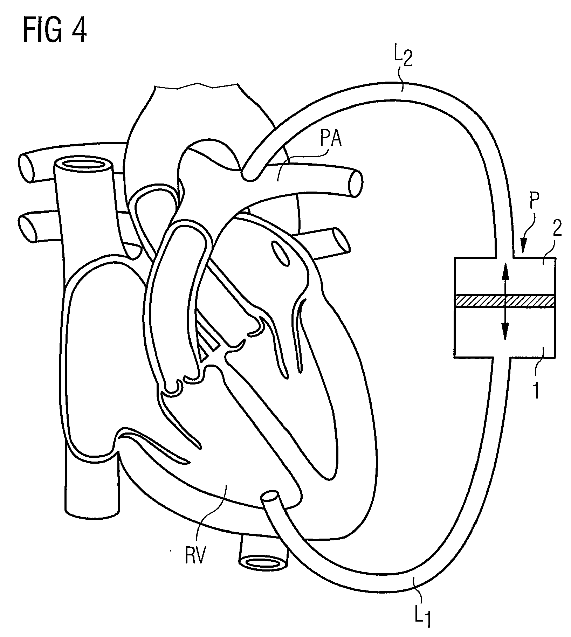

Figur 4 zeigt schließlich noch eine weitere Abwandlung des in Figur 1 dargestellten grundlegenden Prinzips. Hier sind die Leitungen L₁ und L₂ der Pulsationsblutpumpe nicht mit dem linken Ventrikel LV und der Aorta AO sondern stattdessen mit dem rechten Ventrikel RV und den Pulmonalarterien PA verbunden.

Es besteht auch die Möglichkeit, zwei getrennte Pulsationsblutpumpen der vorbeschriebenen Art einerseits für die linke Herzhälfte und andererseits für die rechte Herzhälfte gleichzeitig zu betreiben.

## Patentansprüche

1. Extravasale Pulsationsblutpumpe, umfassend
- eine erste Leitung (L₁) zur Verbindung der Pulsationsblutpumpe an eine Herzkammer (LV; RV),
- eine zweite Leitung (L₂) zur Verbindung der Pulsationsblutpumpe an eine Blutbahn (AO; PA) und
- ein bidirektionales Pumpsystem (P; P, M),
**dadurch gekennzeichnet, dass** das bidirektionale Pumpsystem dazu eingerichtet ist, abwechselnd einerseits Blut durch die erste Leitung (L₁) anzusaugen und gleichzeitig Blut durch die zweite Leitung (L₂) auszustoßen und andererseits Blut durch die zweite Leitung (L₂) anzusaugen und gleichzeitig Blut durch die erste Leitung (L₁) auszustoßen, wobei eine Steuerung (St) vorgesehen ist, das Pumpsystem (P; P, M) entsprechend einem vorgegebenen Herzrhythmus abwechselnd in die eine und die andere Richtung (R) zu betreiben.

2. Pulsationsblutpumpe nach Anspruch 1, umfassend eine mit der Steuerung (St) gekoppelte Sensorik (S) zum Erfassen und Übermitteln von Herzrhythmusdaten an die Steuerung (St).

3. Pulsationsblutpumpe nach Anspruch 1 oder 2, wobei das Pumpsystem (P; P, M) eine erste Pumpkammer (1) mit variablem Volumen (V₁), die an die erste Leitung (L₁) angeschlossen ist, und eine zweite Pumpkammer (2) mit variablem Volumen (V₂), die an die zweite Leitung (L₂) angeschlossen ist, aufweist, wobei die erste und zweite Pumpkammer (1, 2) so miteinander gekoppelt sind, dass, wenn Blut durch die erste Leitung (L₁) in die erste Pumpkammer (1) gesaugt wird, Blut von der zweiten Pumpkammer (2) in die zweite Leitung (L₂) ausgestoßen wird, und umgekehrt.

4. Pulsationsblutpumpe nach Anspruch 3, wobei die erste Pumpkammer (1) mit einer ersten Compliancekammer (C₁) mit variablem Volumen eine erste Doppelkammer und die zweite Pumpkammer (2) mit einer zweiten Compliancekammer (C₂) mit variablem Volumen eine zweite Doppelkammer bilden, wobei die erste Pumpkammer (1) von der ersten Compliancekammer (C₁) und die zweite Pumpkammer (2) von der zweiten Compliancekammer (C₂) jeweils durch eine variable Trennwand (M₁, M₂) getrennt sind, und wobei das Pumpsystem eine Pumpe (P) umfasst, die dazu eingereicht ist, ein Fluid zwischen der ersten Compliancekammer (C₁) und der zweiten Compliancekammer (C₂) hin und her zu fördern.

5. Pulsationsblutpumpe nach Anspruch 4, wobei die Trennwände (M₁, M₂) jeweils eine flexible Membran umfassen.

6. Pulsationsblutpumpe nach einem der Ansprüche 4 oder 5, wobei das Fluid eine Flüssigkeit ist.

7. Pulsationspumpe nach einem der Ansprüche 1 bis 6, wobei das Pumpsystem ein Differentialpumpsystem ist.

8. Pulsationsblutpumpe nach einem der Ansprüche 1 bis 7, wobei das Pumpsystem (P; P, M) eingerichtet ist, in den Körper eines Patienten implantiert zu werden.

## Claims

1. An extravascular pulsation blood pump, comprising
- a first line (L₁) for connecting the pulsation blood pump to a heart ventricle (LV; RV),
- a second line (L₂) for connecting the pulsation blood pump to a bloodstream (AO; PA), and
- a two-way pumping system (P; P, M),
**characterized in that** the two-way pumping system is adapted to alternately on the one hand suck in blood through the first line (L₁) and simultaneously expel blood through the second line (L₂), and on the other hand suck in blood through the second line (L₂) and simultaneously expel blood through the first line (L₁), wherein a controller (St) is provided for operating the pumping system (P; P, M) in accordance with a predetermined heart rhythm alternately in the one and the other direction (R).

2. The pulsation blood pump according to claim 1, comprising sensors (S) coupled to the controller (St) for capturing and transmitting heart rhythm data to the controller (St).

3. The pulsation blood pump according to claim 1 or 2, wherein the pumping system (P; P, M) has a first pump chamber (1) with variable volume (V₁) which is connected to the first line (L₁), and a second pump chamber (2) with variable volume (V₂) which is connected to the second line (L₂), wherein the first and second pump chamber (1,2) are so coupled to each other, that, when blood is sucked through the first line (L₁) into the pump chamber (1), blood is expelled from the second pump chamber (2) into the second line (L₂), and vice versa.

4. The pulsation blood pump according to claim 3, wherein the first pump chamber (1) with a first compliance chamber (C₁) with variable volume form a first double chamber, and the second pump chamber (2) with a second compliance chamber (C₂) with variable volume form a second double chamber, wherein the first pump chamber (1) is separated from the first compliance chamber (C₁) and the second pump chamber (2) is separated from the second compliance chamber (C₂) by a variable partition (M₁, M₂) in each case, and wherein the pumping system comprises a pump (P) comprises which is adapted to convey a fluid back and forth between the first compliance chamber (C₁) and the second compliance chamber (C₂).

5. The pulsation blood pump according to claim 4, wherein the partitions (M₁, M₂) each comprise a flexible membrane.

6. The pulsation blood pump according to any of the claims 4 or 5, wherein the fluid is a liquid.

7. The pulsation blood pump according to any of the claims 1 to 6, wherein the pumping system is a differential pumping system.

8. The pulsation blood pump according to any of the claims 1 to 7, wherein the pumping system (P; P, M) is adapted to be implanted in a patient's body.

## Revendications

1. Pompe à sang à pulsations extra-vasculaire, comprenant
une première conduite (L₁) pour la connexion de la pompe à sang à pulsations à un ventricule (LV; RV),
une deuxième conduite (L₂) pour la connexion de la pompe à sang à pulsations à un circuit sanguin (AO; PA), et
un système de pompage (P; P , M) bidirectionnel,
**caractérisé en ce que** le système de pompage bidirectionnel est conçu pour, en alternance, d'une part aspirer du sang par la première conduite (L₁) et propulser en même temps du sang par la deuxième conduite (L₂), et d'autre part aspirer du sang par la seconde conduite (L₂) et propulser en même temps du sang par la première conduite (L₁), une commande (St) étant prévue pour actionner le système de pompage (P; P , M), en correspondance avec un rythme cardiaque prédéterminé, en alternance dans l'une et dans l'autre direction (R).

2. Pompe à sang à pulsations selon la revendication 1, comprenant un ensemble de capteurs (S) couplés à la commande (St) pour la saisie et le transmission de données de rythme cardiaque à la commande (St).

3. Pompe à sang à pulsations selon la revendication 1 ou 2, le système de pompage (P; P , M) comportant une première chambre de pompage (1) à volume (V₁) variable raccordée à la première conduite (L₁), et une deuxième chambre de pompage (2) à volume (V₂) variable raccordée à la deuxième conduite (L₂), la première et la deuxième chambre de pompage (1, 2) étant couplées de telle manière entre elles que, quand du sang est aspiré par la première conduite (L₁) vers la première chambre de pompage (1), du sang de la deuxième chambre de pompage (2) est propulsé dans la deuxième conduite (L₂), et inversement.

4. Pompe à sang à pulsations selon la revendication 3, la première chambre de pompage (1) formant avec une première chambre de compliance (C₁) à volume variable une première chambre double, et la deuxième chambre de pompage (2) formant avec une deuxième chambre de compliance (C₂) à volume variable une deuxième chambre double , une paroi de séparation variable (M₁, M₂) séparant respectivement la première chambre de pompage (1) de la première chambre de compliance (C₁) et la deuxième chambre de pompage (2) de la deuxième chambre de compliance (C₂), et le système de pompage comprenant une pompe (P) conçue pour véhiculer en va-et-vient un fluide entre la première chambre de compliance (C₁) et la deuxième chambre de compliance (C₂).

5. Pompe à sang à pulsations selon la revendication 4, les parois de séparation (M₁, M₂) comprenant respectivement une membrane flexible.

6. Pompe à sang à pulsations selon une des revendications 4 ou 5, le fluide étant un liquide.

7. Pompe à pulsations selon une des revendications de 1 à 6, le système de pompage étant un système de pompage différentiel.

8. Pompe à sang à pulsations selon une des revendications de 1 à 7, le système de pompage (P; P , M) étant conçu pour être implanté dans le corps d'un patient.
